# EUROPEAN PATENT APPLICATION

(11) **EP 1 911 840 A1**
(43) Date of publication of application: **16.04.2008**
(21) Application number: 06782196.7
(22) Date of filing: 02.08.2006
(51) Int. Cl.: C12N 15/00, C12N 15/09, C12P 21/00

(54) **tRNA SYNTHESIS METHOD, NUCLEIC ACID, AMINOACYL tRNA SYNTHESIS METHOD, AND PROCESS FOR PRODUCTION OF PROTEIN HAVING NON-NATURAL AMINO ACID INTEGRATED THEREIN**

(30) Priority: 02.08.2005 JP 2005224638
(71) Applicant: Riken, Wako-shi, Saitama 351-0198 (JP)
(72) Inventor: YOKOYAMA, Shigeyuki, c/o RIKEN Yokohama Institute, Yokohama-shi, Kanagawa 230-0045 (JP); SAKAMOTO, Kensaku, c/o RIKEN Yokohama Institute, Yokohama-shi, Kanagawa 230-0045 (JP); YANAGISAWA, Tatsuo, c/o RIKEN Yokohama Institute, Yokohama-shi, Kanagawa 230-0045 (JP); KOBAYASHI, Takatsugu, c/o RIKEN Yokohama Institute, Yokohama-shi, Kanagawa 230-0045 (JP)
(74) Representative: Williams, Gareth Owen
(86) International application number: PCT/JP2006/315327
(87) International publication number: WO 2007/015527

(57) **Abstract**

The present invention relates to a process for producing a protein having an unnatural amino acid introduced therein, the process including: expressing in a eukaryotic cell an aminoacyl-tRNA synthetase, a nucleic acid having a sequence containing a eukaryote-derived tRNA nucleotide sequence linked to the 5' end of a tRNA nucleotide sequence that is ligated with to an unnatural amino acid in the presence of the aminoacyl-tRNA synthetase, an unnatural amino acid, and a gene of a desired protein having a nonsense mutation at a predetermined position, to integrate the unnatural amino acid at the nonsense mutation position into the protein, thereby expressing a protein having an unnatural amino acid introduced therein.

## Description

### TECHNICAL FIELD

The present invention relates to a tRNA synthesis method, a nucleic acid, an aminoacyl-tRNA synthesis method, and a process for producing a protein having an unnatural amino acid introduced therein.
Priority is claimed on Japanese Patent Application No. 2005-224638, filed August 2, 2005, the contents of which are incorporated herein by reference.

### BACKGROUND ART

Proteins introduced with unnatural amino acids (hereunder, referred to as "alloproteins") in which an amino acid residue in a predetermined position thereof is replaced with an amino acid other than the 20 types of amino acids involved in common protein synthesis (an unnatural amino acid), can be an effective tool for the functional/structural analysis of proteins. Lysine derivatives include amino acids synthesized by posttranslational modifications, such as acetyllysine and methyllysine. They are famous for their association with the regulation of gene expression particularly in histones, but are also known to be associated with the regulation of transcriptional activation, the regulation of protein-protein interactions, and the suppression/enhancement of ubiquitination of many other proteins. Site-specific incorporation of such lysine derivatives into eukaryotes is expected to lead many discoveries about lysine acetylation, methylation, and so forth.

Pyrrolysyl-tRNA synthetase (PylRS) refers to a novel aminoacyl-tRNA synthetase (aaRS) discovered from methanogenic archaea (the genus *Methanosarcina*). The corresponding tRNA (pyrrolysine-tRNA) is a suppressor tRNA, which has a unique secondary structure with an unusually small D-loop. Recently, it was found that PylRS and pyrrolysine-tRNA do not interact with endogenous aaRS and tRNA (orthogonality) and can incorporate pyrrolysine into proteins in an amber codon-specific manner in *E. coli* (Non Patent Document 1). Moreover, the wild type PyIRS was found to be capable of binding unnatural amino acids such as Ns-Boc-L-lysine to pyrrolysine-tRNA in *E. coli* (Non Patent Document 1).
[Non Patent Document 1] S. K. Blight, et al., Nature, 431, 333-335 (2004)

### DISCLOSURE OF INVENTION

### [Problems to be Solved by the Invention]

However, it has not been discovered yet whether or not PylRS and pyrrolysine-tRNA have orthogonality in eukaryotes, particularly in animal cells, and whether these can be used for expanding genetic codes.

Accordingly, an object of the present invention is to provide a process for producing a protein having an unnatural amino acid introduced therein in a eukaryotic, using an aminoacyl-tRNA synthetase and an aminoacyl-tRNA.
Another object of the present invention is to provide a method for synthesizing such an aminoacyl-tRNA in a eukaryotic cell.
Yet another object of the present invention is to provide a method for synthesizing a tRNA in a eukaryotic cell.
Another object of the present invention is to provide a nucleic acid for synthesizing such a tRNA in a eukaryotic cell.

### [Means for Solving the Problem]

The present inventors have conducted through research to achieve the above objectives, and have focused on promoters. In general, the expression of tRNA in eukaryotic cells requires two internal promoters within the tRNA encoding sequence, and the consensus sequences thereof are known as box A and box B. For example, although the suppressor tyrosine tRNA of *Bacillus stearothermophilus* is derived from prokaryotes, the box A and the box B are present in the suppressor tyrosine tRNA sequence (M. Sprinzl et al., Nucleic Acids Research 17, 1-172 (1989)). Thus, the suppressor tyrosine tRNA can be expressed in animal cells without modification. Therefore, if a prokaryote having neither box A nor box B is employed, the introduction of the box A and the box B thereinto is considered to be a must.

However, as described above, the D-loop of a methanogenic archaea-derived pyrrolysine-tRNA is so unusually small with a few base deletions that the introduction of the box A and the box B therein would destroy its functions. Accordingly, even the use of a pyrrolysine-tRNA introduced with the box A and the box B is not capable of synthesizing an alloprotein incorporated with a lysine derivative. Therefore, as a result of further investigation, the present inventors have found that the linkage of a eukaryotic tRNA to the 5' end of the pyrrolysine-tRNA as a promoter enables an effective expression of a methanogenic archaea-derived pyrrolysine-tRNA in animal cells, which has led to the completion of the present invention.

That is, the present invention provides a tRNA synthesis method, including: transcribing in a eukaryotic cell a nucleic acid having a sequence containing a tRNA nucleotide sequence to be synthesized and a eukaryote-derived tRNA nucleotide sequence linked to the 5' end of the tRNA nucleotide sequence.
In addition, the present invention provides a nucleic acid used for such a tRNA synthesis method, wherein the nucleic acid has a sequence containing a tRNA nucleotide sequence and a eukaryote-derived tRNA nucleotide sequence linked to the 5' end of the tRNA nucleotide sequence.
The present invention also provides an aminoacyl-tRNA synthesis method, including: transcribing in a eukaryotic cell, a nucleic acid having a sequence containing a tRNA nucleotide sequence and a eukaryote-derived tRNA nucleotide sequence linked to the 5' end of the tRNA nucleotide sequence, in the presence of an aminoacyl-tRNA synthetase corresponding to the tRNA.
The present invention provides a process for producing a protein having an unnatural amino acid introduced therein, including: expressing in a eukaryotic cell: an aminoacyl-tRNA synthetase, a nucleic acid having a sequence containing a tRNA nucleotide sequence and a eukaryote-derived tRNA nucleotide sequence linked to the 5' end of the tRNA nucleotide sequence that can bind to an unnatural amino acid in the presence of the aminoacyl-tRNA synthetase, the unnatural amino acid, and a gene of a desired protein having a nonsense mutation at a predetermined position, to integrate the unnatural amino acid at the nonsense mutation position in the protein, thereby expressing a protein having an unnatural amino acid introduced therein.

### [Effects of the Invention]

The use of the production process according to the present invention enables effective expressions of a tRNA and an aminoacyl-tRNA in eukaryotic cells, and makes it possible to express a tRNA free from boxes A and B in the eukaryotic cells.
Moreover, the use of the production process according to the present invention enables the synthesis of alloproteins introduced with a lysine derivative, particularly, such as Nε-acetyllysine and Nε-trimethyllysine, which exist in eukaryotes, or Nε-2-methylamino-benzoyllysine containing a fluorescent group, using a wild type aminoacyl-tRNA synthetase.

### BRIEF DESCRIPTION OF THE DRAWINGS

FIG. 1 illustrates a cloverleaf structure of pyrrolysine-tRNA.
FIG. 2 shows results of Grb2 (111 amb) suppression in Western blotting analysis in Example 1.
FIG. 3 is mass spectrum data showing that Nε-Boc-lysine was incorporated into peptides in the presence of PylRS and pyrrolysine-tRNA in *E. coli.*
FIG. 4 illustrates a cloverleaf structure of pyrrolysine-tRNA introduced with box A and box B.
FIG. 5 shows results of Grb2 (111 amb) suppression in Western blotting analysis in Comparative Example 1.
FIG. 6 shows an *in vitro* experiment on whether or not each lysine derivative was ligated to a pyrrolysine-tRNA by purified PylRS.

### BEST MODE FOR CARRYING OUT THE INVENTION

### (Unnatural amino acid)

It is particularly preferable that an unnatural amino acid used in the present invention is a lysine derivative. The lysine derivative is preferably an unnatural amino acid in which a hydrogen atom bonded to a nitrogen atom in the ε-position is substituted with another atom or atomic group. Examples thereof include pyrrolysine, Nε-t-butoxycarbonyllysine (Nε-Boc-lysine), Nε-acetyllysine, Nε-trimethyllysine, and Nε-2-methylamino-benzoyllysine (Nma-lysine). In particular, the introduction of methyllysine or acetyllysine, which are modified lysines existing in eukaryotes, into proteins in a site-specific manner would possibly provide many findings on the lysine acetylation and methylation. Alloproteins introduced with these lysine derivatives are also useful as materials for the functional/structural analysis of proteins, and might be a target of drug discovery.

### (Aminoacyl tRNA synthetase)

The aminoacyl-tRNA synthetase used in the present invention is a tRNA synthetase which is capable of recognizing an unnatural amino acid, and specifically recognizing an aminoacyl-tRNA, so as to produce a suppressor tRNA charged with the unnatural amino acid.
It is preferable that such a tRNA synthetase be a methanogenic archaea-derived PylRS which is capable of recognizing a lysine derivative as an amino acid, and specifically recognizing a pyrrolysine-tRNA used in combination as tRNA, so as to produce a suppressor tRNA bonded with the lysine derivative. As the methanogenic archaea, *Methanosarcina mazei (M. mazei)* is preferred.

PylRS is expressed in eukaryotic cells, preferably in animal cells, and particularly preferably in mammalian cells. In order to express PylRS in mammalian cells, for example, a DNA sequence constructed by adding a FLAG tag or the like to the N-terminus region of a wild type gene derived from the *Methanosarcina mazei* is amplified by a PCR method, the PCR products are inserted into the NheI-BamHI site of one of the commercially available pcDNA3.1 (manufactured by Invitrogen), pAGE107 (Cytotechnology, 3, 133 (1990)), pAGE103 (J. Biochem. 101, 1307 (1987)), and so forth, and the thus constructed plasmid may be transferred into mammalian cells.
Examples of the method for transferring a vector into cells include the electroporation method (Nucleic, Acids Res. 15, 1311-1326 (1987)), the potassium phosphate method (Mol. Cell Biol. 7, 2745-2752 (1987)), the lipofection method (Cell 7, 1025-1037 (1994); Lamb, Nature Genetics 5, 22-30 (1993)), and so forth.

### (tRNA)

The tRNA to be used in combination with the above aminoacyl-tRNA synthetase has an anticodon and a tertiary structure that are complementary to a nonsense codon for functioning as a suppressor tRNA, and is expressed in eukaryotic cells, preferably in animal cells, and particularly preferably in mammalian cells. In cases where the aminoacyl-tRNA synthetase is PyIRS, the corresponding pyrrolysine-tRNA is a tRNA derived from a non-eukaryotic cell which has an anticodon and a tertiary structure that are complementary to a nonsense codon for functioning as a suppressor tRNA, and is expressed in eukaryotic cells. That is, it is a suppressor tRNA which satisfies requirements; of being allotted to a nonsense codon differing from any codon allotted to 20 types of common amino acids, of being recognized only by the lysine derivative-specific PyIRS, and of not being recognized by normal host aaRS (orthogonality), and is expressed in animal cells.
Here, examples of the nonsense codon include UAG (amber), UAA (ochre), and UGA (opal), although a UAG (amber) codon is preferably used.

As described above, the expression of tRNA in eukaryotic cells requires two internal promoters within the tRNA encoding sequence, and the consensus sequences thereof are known as box A and box B.

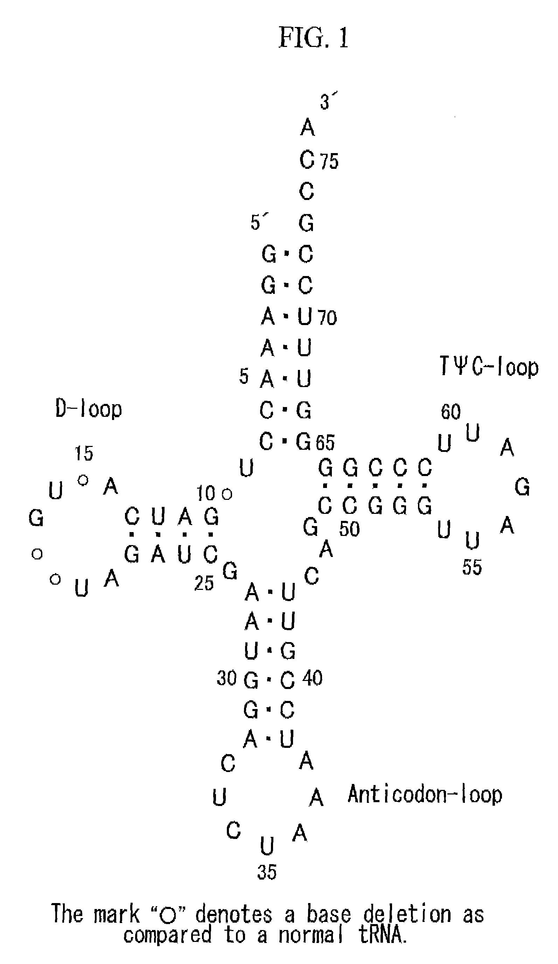

FIG. 1 shows a cloverleaf structure of pyrrolysine-tRNA. In FIG. 1, the mark "○" denotes a base deletion in the loop on the left (D-loop). In this manner, the pyrrolysine-tRNA has three base deletions in the D-loop, which is unusually smaller than D-loops of other tRNAs. Even if the box A and box B sequences are introduced into the pyrrolysine-tRNA in order to express the pyrrolysine-tRNA in animal cells, the structure of the pyrrolysine-tRNA is conventionally changed significantly due to the unusually small D-loop of the pyrrolysine-tRNA, which results in the disabling of the suppressor activity maintenace.

### (Synthesis of tRNA and aminoacyl-tRNA)

In the aminoacyl-tRNA synthesis method of the present invention, a eukaryote-derived tRNA is linked to the 5' end of the wild type tRNA to effect the transcription in eukaryotic cells, preferably in animal cells, and particularly preferably in mammalian cells, in the presence of an aminoacyl-tRNA synthetase. At this time, a transcription termination sequence is preferably linked to the 3' end of the tRNA. More specifically, a sequence of *Methanosarcina mazei*-derived wild type pyrrolysine-tRNA having the 5' end linked with a eukaryote-derived tRNA and the 3' end linked with a transcription termination sequence, is first synthesized using DNA primers, then is incorporated into, for example, pCR4Blunt-TOPO (manufactured by Invitrogen), and the thus constructed plasmid is transferred into animal cells to thereby effect the expression, followed by the transcription in the animal cells for processing. At this time, the 5' end of the wild type pyrrolysine-tRNA and the eukaryote-derived tRNA are preferably linked via a linker. There is no particular limitation on the linker, and examples thereof include those cleavable with BglII, XbaI, XhoI, or the like. The tRNA to be linked to the 5' end is derived from a eukaryote, and there is no particular limitation on the eukaryote, examples of which include animals, plants, and insects. Among them, human-derived tRNA is preferred. There is also no particular limitation on the amino acid to be linked with the tRNA as long as it is selected from 20 types of common natural amino acids. Among them, valine is preferred.

In the tRNA synthesis method according to the present invention, a eukaryote-derived tRNA is linked to the 5' end of the tRNA to be synthesized to effect the transcription in eukaryotic cells. The tRNA to be synthesized is not specifically limited, and refers to all types of tRNA, examples of which include eukaryote-derived tRNA (including eukaryotic mitochondrial tRNA) and prokaryote-derived tRNA. The tRNA is preferably derived from prokaryotes such as eubacteria and archaebacteria, and more preferably a pyrrolysine tRNA. In addition, the 3' end thereof is preferably linked with a transcription termination sequence. The eukaryotic cells are preferably animal cells, and particularly preferably mammalian cells. The eukaryote-derived tRNA is, for example, derived from animals, plants, and insects. Among them, a human-derived tRNA is preferred. There is no particular limitation on the amino acid to be linked with the tRNA as long as it is selected from the 20 types of common natural amino acids. Among them, valine is preferred.

### (Proteins to be incorporated with unnatural amino acid)

The types of proteins to be incorporated with unnatural amino acids according to the present invention are not limited, and any expressible protein, even a heterologous recombinant protein, may be employed. Examples of such protein types include so-called signal transduction related proteins, receptors, growth factors, cell cycle related factors, transcriptional factors, translational factors, transport related proteins, secretory proteins, cytoskeleton related proteins, enzymes, chaperons, and disease related proteins for cancer, diabetes, hereditary diseases, and the like.

In the present invention, it is necessary to introduce a nonsense codon (an amber codon if the suppressor tRNA is an amber suppressor) into position to be incorporated with an unnatural amino acid, in particular, a lysine derivative. By so doing, an unnatural amino acid, in particular, a lysine derivative, can be specifically incorporated into the nonsense codon (amber codon) site.

Commonly known methods may be employed to introduce a mutation into a protein in a site-specif manner. Although there is no particular limitation, it can be appropriately carried out in accordance with methods described in Gene 152, 271-275 (1995), Methods Enzymol., 100, 468-500 (1983), Nucleic Acids Res., 12, 9441-9456 (1984), Proc. Natl. Acad. Sci. U.S.A., 82, 488-492 (1985), and "Cell Technology, extra number, New Cell Technology Experimental Protocol, Shujunsha, pp. 241-248 (1993)", methods using the "Quick Change Site-Directed Mutagenesis Kit" (manufactured by Stratagene), or the like.
Since the present invention is capable of expression in animal cells, an unnatural amino acid can be incorporated in proteins which have been so far unable to be expressed, able to be merely expressed at a low level, or unable to receive posttranslational modifications to be in the active form, in *E. coli* or cell-free protein systems. Various types of such proteins are known among those skilled in the art.
For example, alloproteins of an extracellular domain of tyrosine kinase-type receptor such as human EGFR (Cell, 110, 775-787 (2002)), a human Croucho/TLE1 protein (Structure 10, 751-761 (2002)), a rat muscle-specific kinase (Structure 10. 1187-1196 (2002)), and the like, can be synthesized, but the proteins are not limited thereto.
Moreover, in the method of the present invention, since alloproteins are expressed in animal cells, glycoproteins in which sugar chains are bonded can be incorporated with an unnatural amino acid, particularly a lysine derivative. Particularly, in cases where a type of glycoprotein whose glycosylation pattern in a cell-free protein system is different from its original pattern, the system in animal cells according to the present invention can be considered to be an effective system for obtaining alloproteins including added sugar chains of the objective (original) pattern.

Proteins to be incorporated with an unnatural amino acid, in particular a lysine derivative, can be expressed such that, for example, a gene comprising a sequence constructed so that codons corresponding to desired amino acids of a desired protein are replaced with nonsense codons and which a desired tag is added to the C-terminus thereof, is incorporated into the BamHI-XhoI site of pcDNA4/TO or the like, and the thus constructed plasmid is transferred into animal cells.

### (Host)

Host animal cells utilized for the present invention are preferably mammalian cells of which the gene recombination system has been established. Examples of useful mammalian host cell lines include Chinese hamster ovary (CHO) cells and COS cells. More specific examples thereof include SV40-transformed monkey kidney CV-1 cell lines (COS-7, ATCC CRL 1651); human embryonic kidney cell lines (293 cells or 293 cells subcloned for proliferation in a suspension culture, J. Gen Virol., 36: 59 (1977)); Chinese hamster ovary cells/-DHFR (CHO, Proc. Natl. Acad. Sci. USA, 77: 4216 (1980)); mouse Sertoli cells (TM4, Biol. Reprod., 23: 243-251 (1980)); human lung cells (W138, ATCC CCL 75); human liver cells (Hep G2, HB 8065); and mouse mammary tumor (MMT 060562, ATCC CCL51). The expression systems of these hosts have each been established, and the selection of an appropriate host cell is within the technical scope of those skilled in the art.
These can be performed in accordance with methods described in, for example, Molecular Cloning, Third Edition, Cold Spring Harbor Laboratory Press (2001), and the like.

The incorporation of the above three types of plasmids into animal cells to effect expression enables the expression of an alloprotein in which the target lysine derivative is incorporated.
In other words, animal cells having: (A) an expression vector that expresses an aminoacyl-tRNA synthetase, particularly a PyIRS, in animal cells; (B) an expression vector that expresses a *Methanosarcina mazei*-derived pyrrolysine-tRNA that is bindable to an unnatural amino acid, in particular, a lysine derivative, in the presence of the aminoacyl-tRNA synthetase, particularly, a PylRS, in the animal cells; (C) an expression vector that expresses a desired protein having a nonsense mutation at a predetermined position; and an unnatural amino acid, particularly, a lysine derivative, are incubated in a suitable medium for the animal cell growth (such as, for example, Opti-MEM I (Gibco BRL) in the case of CHO cells) under conditions appropriate for proliferation of the animal cells. For example, in the case of CHO cells, the incubation is performed at about 37°C for about 24 hours.

Next, the present invention will be explained in more detail with reference to examples. However, the present invention is not limited to these examples.

### [Example 1]

Grb2 is a protein which intracellularly interacts with epidermal growth factor receptors and is involved in carcinogenesis of cells. In the present Example, an experiment was performed in which a lysine derivative was incorporated into the position 111 of human Grb2.

### (Construction of a PylRS expression plasmid)

A DNA sequence (SEQ ID: NO 1) constructed so that a FLAG tag was added to the N-terminus region of a *Methanosarcina mazei*-derived wild type PylRS gene was amplified by the PCR method. The PCR products were introduced into the NheI-BamHI site of pcDNA3.1 to construct a plasmid.

### (Construction of a pyrrolysine-tRNA expression plasmid)

A sequence (SEQ ID: NO 3) in which a human valine tRNA was linked to the 5' end of a *Methanosarcina mazei*-derived wild type pyrrolysine-tRNA via a linker (SEQ ID: NO 2) and a transcription termination sequence was linked to the 3' end thereofwas synthesized using DNA primers. The resultant product was incorporated into the pCR4Blunt-TOPO to construct a plasmid.

### (Construction of a plasmid that expresses a protein incorporated with a lysine derivative)

The leucine codon at the position 111 of human grb2 was converted into an amber codon (grb2 (Am111)) using a Quick Change site-directed mutagenesis kit (Stratagene). Next, a gene (SEQ ID: NO 4) constructed so that the FLAG tag (DYKDDDDK) was added to the C-terminus was incorporated into the BamHI-XhoI site of pcDNA4/TO to construct a plasmid for detecting suppressions.

### (Gene transfection into cells and suppression reaction)

The above three types of plasmids were transfected into 90% confluent Chinese hamster ovary cells (CHO cells, a culture medium containing DMEM/F-12 (Gibco), 10% FBS (ICN), and 1/100 penicillin-streptomycin (Gibco) was used for the passage thereof) that had been cultured in a 2.0 mL culture scale in a 6-well plate, at 0.5 µg per well, in various combinations (refer to the results). The transduction was performed by a method using Lipofectamine 2000 (Invitrogen) according to its instruction manual. Opti-MEM (Gibco) was used as a culture medium for the transduction.
After transduction, the cell culture medium was replaced with DMEM/F-12 (Gibco) with or without 1 mM Boc-lysine (Bachem) to which 1 µg/mL tetracycline was added to induce the expression. The culture solution was then placed in a CO₂ incubator for about 20 hours to continue the culture at 37°C.

### (Detection)

The cell culture medium was removed and the cells were washed with a buffer solution. Then, the cells were lysed, and the proteins thereof were collected. SDS-polyacrylamide gel electrophoresis was performed to separate the proteins by molecular weight, followed by electroblotting on a membrane (100V, 1 hour). An anti-FLAG M2 (Sigma) was used as the primary antibody and a mouse IgG horseradish peroxidase-linked whole antibody from sheep (Amersham) was used as the secondary antibody. ECL Western blotting detection reagent (Amersham) was used as a detection reagent. The measurement was performed using a cooled CCD camera LAS 1000 plus (Fujifilm).

### (Results)

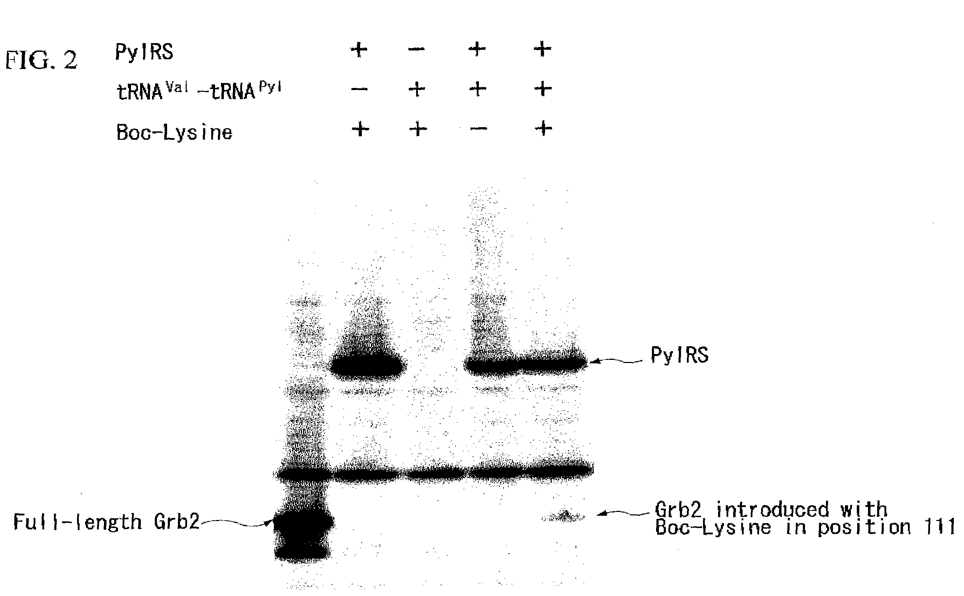

FIG. 2 presents Western blots showing the detection results of Grb2 (111 amb) suppression. The lane 1 on the left is the control for indicating the position of the full-length Grb2 band. The FLAG tag was added to the C-terminus of the wild type Grb2, and if the synthesis was carried out to the C-terminus, a band in the position indicated by the Grb2 arrow is detected. The lanes 2 to 4 show cases where any one of PylRS, pyrrolysine-tRNA, and Nε-Boc-lysine was lacking. In these cases, the full-length of Grb2 was not synthesized. In addition, the lane 5 shows a case where PylRS, pyrrolysine-tRNA, and Nε-Boc-lysine were transferred into cells, and the full-length of Grb2 was synthesized. The above results show that Nε-Boc-lysine was introduced by PylRS and pyrrolysine-tRNA into the amber codon introduced in the position 111 of Grb2.

### (Reference Example 1)

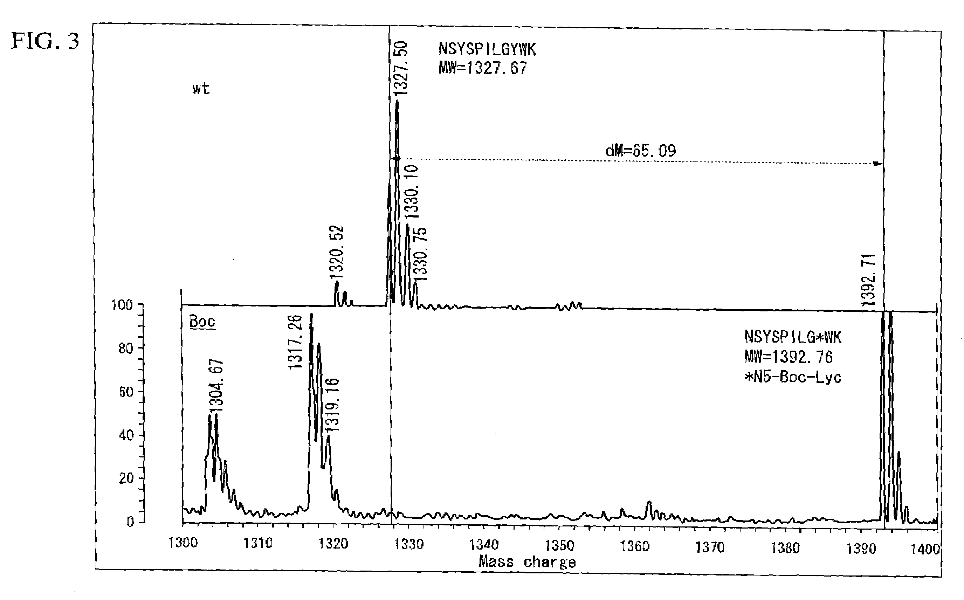

FIG. 3 is mass spectrum data showing that Nε-Boc-lysine was incorporated into peptides in the presence of PylRS and pyrrolysine-tRNA in *E. coli.* In the figure, the peak molecular weight (MW) of 1327.67 indicates a peptide having a sequence of NSYSPILGYWK. The peak molecular weight of 1392.76 indicates a peptide in which the 11th tyrosine from the left was replaced with Nε-Boc-lysine (shown by *).

### (Comparative Example 1)

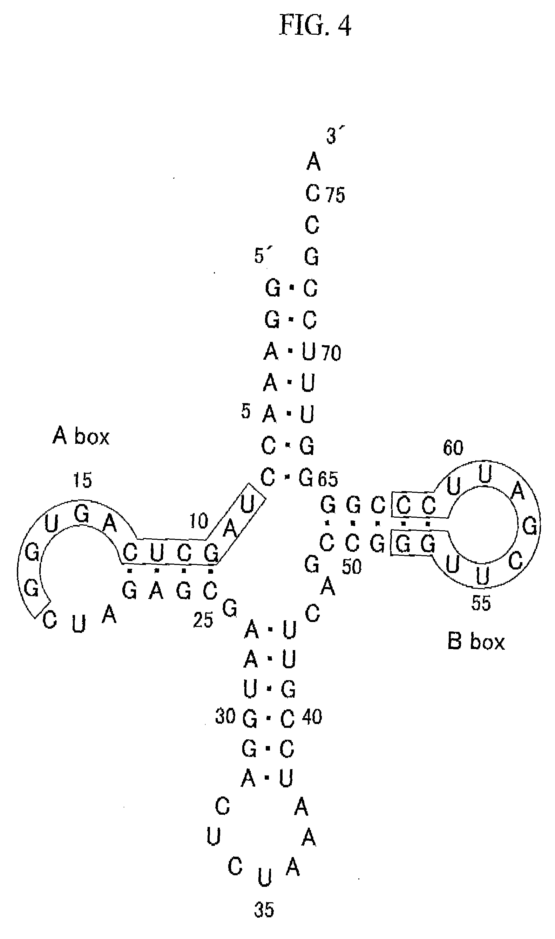

Plasmids were constructed, genes were transferred, and the suppression reaction was detected in the same manner as that of Example 1, except that a construction method in which box A and box B were introduced into the *Methanosarcina mazei*-derived wild type pyrrolysine-tRNA was adopted instead of the construction method of the pyrrolysine-tRNA expression plasmid in the Example 1. FIG. 4 illustrates a cloverleaf structure of the pyrrolysine-tRNA having the introduced boxes A and B. The boxes A and B were introduced in accordance with a conventional method.

### (Results)

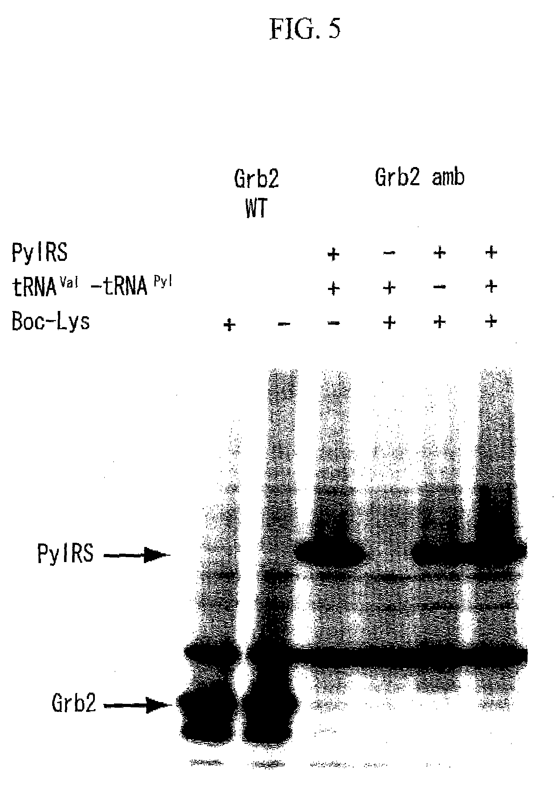

FIG. 5 presents Western blots showing the detection results of Grb2 (111 amb) suppression. The lane 1 on the left is the control for indicating the position of the full-length Grb2 band. The lanes 2 to 4 show cases where any one of PylRS, pyrrolysine-tRNA, and Nε-Boc-lysine was lacking. In these cases, the full-length Grb2 was not synthesized. The lane 5 shows a case where PyIRS, pyrrolysine-tRNA, and Nε-Boc-lysine were all transferred into cells, although the full-length Grb2 was not synthesized. The above results show that, even if box A and box B were introduced into the pyrrolysine-tRNA, the pyrrolysine-tRNA was not expressed, thus the position 111 of Grb2 was not introduced with Nε-Boc-lysine.

### (Reference Example 2)

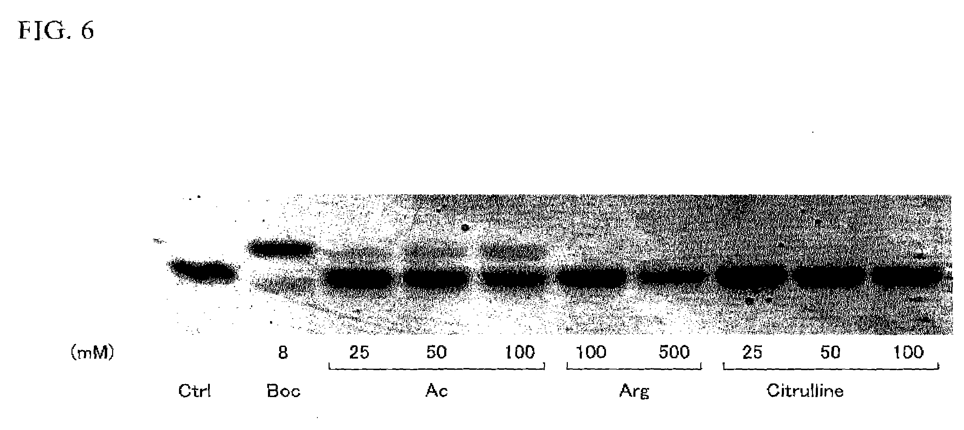

FIG. 6 shows an *in vitro* examination on whether or not respective lysine derivatives are bindable to the pyrrolysine-tRNA in the presence of purified PyIRS and purified pyrrolysine-tRNA. The bands shifted upwards indicate those bound to pyrrolysine-tRNA. Boc denotes Nε-Boc-lysine, Ac denotes Nε-acetyllysine, and Arg denotes arginine. In FIG. 6, Nε-Boc-lysine was 8 mM, in which case it was bound to the pyrrolysine-tRNA. However, even if the Nε-Boc-lysine was 1 mM or less, it was confirmed that PylRS was capable of binding the Nε-Boc-lysine to the pyrrolysine-tRNA.

### INDUSTRIAL APPLICABILITY

The present invention can be effectively used for transferring lysine derivatives to synthesize alloproteins.

## Claims

1. A tRNA synthesis method, comprising:
transcribing in a eukaryotic cell a nucleic acid having a sequence comprising a tRNA nucleotide sequence to be synthesized and a eukaryote-derived tRNA nucleotide sequence linked to a 5' end of the tRNA nucleotide sequence to be synthesized.

2. The tRNA synthesis method according to claim 1, wherein the tRNA to be synthesized is a noneukaryote-derived tRNA.

3. The tRNA synthesis method according to claim 2, wherein the noneukaryote-derived tRNA is a pyrrolysine-tRNA.

4. The tRNA synthesis method according to any one of claims 1 to 3, wherein the nucleic acid further comprises a transcription termination sequence linked to a 3' end of the tRNA nucleotide sequence to be synthesized.

5. The tRNA synthesis method according to any one of claims 1 to 4, wherein the eukaryotic cell is an animal cell.

6. The tRNA synthesis method according to claim 5, wherein the animal cell is a mammalian cell.

7. The tRNA synthesis method according to any one of claims 1 to 6, wherein the eukaryote-derived tRNA nucleotide sequence is a human valine tRNA nucleotide sequence.

8. A nucleic acid used in the tRNA synthesis method of any one of claims 1 to 7, wherein the nucleic acid has a sequence comprising a tRNA nucleotide sequence and a eukaryote-derived tRNA nucleotide sequence linked to a 5' end of the tRNA nucleotide sequence.

9. An aminoacyl-tRNA synthesis method, comprising:
transcribing in a eukaryotic cell a nucleic acid having a sequence comprising a tRNA nucleotide sequence and a eukaryote-derived tRNA nucleotide sequence linked to a 5' end of the tRNA nucleotide sequence, in a presence of an aminoacyl-tRNA synthetase corresponding to the tRNA.

10. The aminoacyl-tRNA synthesis method according to claim 9, wherein the tRNA is a noneukaryote-derived tRNA.

11. The aminoacyl-tRNA synthesis method according to claim 10, wherein the noneukaryote-derived tRNA is a pyrrolysine-tRNA.

12. The aminoacyl-tRNA synthesis method according to any one of claims 9 to 11, wherein the nucleic acid further comprises a transcription termination sequence linked to a 3' end of the tRNA nucleotide sequence to be synthesized.

13. The aminoacyl-tRNA synthesis method according to any one of claims 9 to 12, wherein the eukaryotic cell is an animal cell.

14. The aminoacyl-tRNA synthesis method according to claim 13, wherein the animal cell is a mammalian cell.

15. The aminoacyl-tRNA synthesis method according to any one of claims 9 to 14, wherein the eukaryote-derived tRNA nucleotide sequence is a human valine tRNA nucleotide sequence.

16. A process for producing a protein having an unnatural amino acid introduced therein, the process comprising:
expressing in a eukaryotic cell an aminoacyl-tRNA synthetase, a nucleic acid having a sequence comprising a tRNA nucleotide sequence and a eukaryote-derived tRNA nucleotide sequence linked to a 5' end of the tRNA nucleotide sequence that is bindable to an unnatural amino acid in a presence of the aminoacyl-tRNA synthetase, the unnatural amino acid, and a gene of a desired protein having a nonsense mutation at a predetermined position, to integrate the unnatural amino acid at the nonsense mutation position in the protein, thereby expressing a protein having an unnatural amino acid introduced therein.

17. The process for producing a protein having an unnatural amino acid introduced therein according to claim 16, wherein the tRNA is a noneukaryote-derived tRNA.

18. The process for producing a protein having an unnatural amino acid introduced therein according to claim 17, wherein the noneukaryote-derived tRNA is a pyrrolysine-tRNA.

19. The process for producing a protein having an unnatural amino acid introduced therein according to any one of claims 16 to 18, wherein the nucleic acid further comprises a transcription termination sequence linked to a 3' end of the tRNA nucleotide sequence.

20. The process for producing a protein having an unnatural amino acid introduced therein according to any one of claims 16 to 19, wherein the eukaryotic cell is an animal cell.

21. The process for producing a protein having an unnatural amino acid introduced therein according to claim 20, wherein the animal cell is a mammalian cell.

22. The process for producing a protein having an unnatural amino acid introduced therein according to any one of claims 16 to 21, wherein the eukaryote-derived tRNA nucleotide sequence is a human valine tRNA nucleotide sequence.

23. The process for producing a protein having an unnatural amino acid introduced therein according to any one of claims 16 to 22, wherein the unnatural amino acid is a lysine derivative.

24. The process for producing a protein having an unnatural amino acid introduced therein according to claim 23, wherein the lysine derivative is selected from the group consisting of pyrrolysine, Nε-t-butoxycarbonyllysine, Nε-acetyllysine, Nε-trimethyllysine, and Nε-2-methylamino-benzoyllysine.
